# EUROPÄISCHE PATENTSCHRIFT

(11) **EP 0 580 175 B1**
(45) Veröffentlichungstag und Bekanntmachung des Hinweises auf die Patenterteilung: **10.05.2000**
(21) Anmeldenummer: 93111882.2
(22) Anmeldetag: 23.07.1993
(51) Int. Cl.: A61N 5/06

(54) **Anordnung zur Ganzkörper-Bräunung**
Arrangement for the tanning of the whole body
Disposition pour le bronzage du corps entier

(30) Priorität: 24.07.1992 DE 4224517
(43) Veröffentlichungstag der Anmeldung: 26.01.1994
(73) Patentinhaber: JK-JOSEF KRATZ GmbH, 53578 Windhagen (DE)
(72) Erfinder: Kratz, Walter, D-53783 Eitorf (DE)
(74) Vertreter: Heim, Hans-Karl, Dipl.-Ing.

(56) Entgegenhaltungen:
- FR-A- 2 585 957

## Beschreibung

Die Erfindung betrifft eine Anordnung zur Ganzkörper-Bräunung.

Bräunungsgeräte auf der Basis von UV-Strahlern sind in unterschiedlichsten Ausbildungen und Größen bekannt. Besonderer Beliebtheit erfreuen sich Bräunungsgeräte zur Ganzkörper-Bräunung. Diese weisen als Unterteil eine Bank auf, die wie ein Oberteil, das im Abstand zur Bank an einem Stativ oder auch an einer Deckenabhängung befestigt sein kann, mit UV-Strahlern bestückt ist. Üblicherweise ist das Oberteil höhenverstellbar, so daß individuelle Bräunungswünsche erfüllt werden können. Eine um die Längsachse schwenkbare Anordnung des Oberteils ermöglicht einen bequemen Einstieg in ein Bräunungsgerät.

Bekannt sind darüberhinaus Sonnenstudios, in denen insbesondere Ganzkörper-Bräunungsgeräte einer gewerblichen Nutzung zugeführt werden. Aufgrund einer großen Nachfrage werden diese Sonnen- oder Bräunungsstudios zunehmend in mittleren bis sehr guten Stadtteilen und Wohnanlagen eingerichtet, in denen über dem Druchschnitt liegende Mietpreise üblich sind.

In Abhängigkeit von der Anzahl der aufgestellten Ganzkörper-Bräunungsgeräte ist auch wegen der zusätzlichen technischen Installationen und wegen der Umkleideräume oder Umkleidekabinen für ein Sonnenstudio ein hinreichend repräsentativer und großer Raum erforderlich. Die Mietkosten erhöhen die gesamten Betriebskosten und wirken sich sowohl für den Betreiber als auch für die Benutzer eines derartigen Sonnenstudios aus.

Aus der nachveröffentlichten DE 42 28 820 A1 ist eine Vorrichtung mit einem klimatisierten Ganzkörper-Bestrahlungsgerät bekannt, welches in einer geschlossenen, begehbaren Kabine mit einem regulierbaren Klimatisierungsgerät angeordnet ist. Das Bestrahlungsgerät weist eine Liege und einen Deckel auf, welcher mit UV-Strahlern versehen ist. Zur Luftzu- und -abführung sind eine Längsseite und die gesamte Decke der Kabine als Luftleitkammern ausgebildet.

Der Erfindung liegt die **Aufgabe** zugrunde, eine besonders gute Rentabilität eines Sonnenstudios zu ermöglichen.

Erfindungsgemäß wird die Aufgabe durch die kennzeichnenden Merkmale des Anspruchs 1 gelöst. Zweckmäßige und vorteilhafte Ausgestaltungen und Weiterbildungen sind in den Unteransprüchen und in der Figurenbeschreibung enthalten.

Ein Grundgedanke der Erfindung ist es, eine effektive Flächennutzung durch eine Einbeziehung der zur Verfügung stehenden Raumhöhe für eine Anordnung von Ganzkörper-Bräunungsgeräten zu erzielen.

Erfindungsgemäß werden wenigstens zwei Ganzkörper-Bräunungsgeräte mehrstöckig, insbesondere übereinander, in einem gemeinsamen Gehäuse angeordnet. Ein erstes Ganzkörper-Bräunungsgerät sollte von einem Fußboden in Normalhöhe aus über eine Einstiegsöffnung zugänglich sein. Zum Einstieg in mindestens ein weiteres Ganzkörper-Bräunungsgerät, welches fluchtend oder auch versetzt über oder unter dem ersten Ganzkörper-Bräunungsgerät angeordnet sein kann, ist eine Zugangsvorrichtung vorgesehen. Die Zugangsvorrichtung dient der Überwindung des Höhenunterschiedes zwischen dem Fußboden und jedem weiteren Ganzkörper-Bräunungsgerät und kann in einer besonders einfachen Ausführung eine Treppe oder ein Podest sein. Erfindungsgemäß weist jedes Ganzkörper-Bräunungsgerät eine separate Einstiegsöffnung auf, welche durch mindestens ein Einstiegselement verschließbar und vorteilhafterweise im Hinblick auf zugehörige Garderobe- oder Umkleideräume bei übereinander angeordneten Geräten zweckmäßigerweise an einander gegenüberliegenden Seitenwänden angeordnet sind.

Für einen besonders bequemen Einstieg ist es sinnvoll, die Einstiegsöffnungen an den Längsseiten der mehrstöckig angeordneten Ganzkörper-Bräunungsgeräte bzw. des gemeinsamen Gehäuses anzuordnen.

Durch die mehrstöckige Anordnung von Ganzkörper-Bräunungsgeräten wird eine hohe Flächenausnutzung bei Sonnenstudios erreicht. So wird beispielsweise für eine Doppelstock-Anordnung nur die halbe Fläche im Vergleich zu der üblichen Aufstellung zweier Ganzkörper-Bräunungsgeräte benötigt, woraus eine erhebliche Mietkostensenkung pro Ganzkörper-Bräunungsgerät resultiert.

In vorteilhafter Weise wird Studiofläche auch deshalb eingespart, weil die Umkleideräume bei einander gegenüberliegenden Einstiegsöffnungen unmittelbar an diese angrenzend angeordnet werden können.

Um den Studioraum möglichst umfassend zu nutzen, kann das gemeinsame Gehäuse, welches schrankartig oder auch gestellähnlich ausgebildet sein kann, bis in den Bereich der Studiodecke reichen.

Bezüglich einer blickdichten Verkleidung des gemeinsamen Gehäuses ist ein Ganzkörper-Bräunungsgerät jeweils aus mehreren Elementen aufgebaut, die in Betriebsstellung eine nahezu geschlossene Bräunungskammer umschließen.

Die einzelnen Elemente umfassen in einer konstruktiv besonders bevorzugten Ausbildung ein Liegenelement, ein parallel angeordnetes Deckenelement sowie seitlich angeordnete Wand- und Einstiegselemente, welche in Längsrichtung mit UV-Strahlern versehen sind und in Betriebsstellung eine allseitige Ganzkörperbräunung ermöglichen.

Es ist außerdem von Vorteil, wenn der Kopfbereich des Dekkenelementes und gegebenenfalls eines jeweils angrenzenden Wandelementes und eines Einstiegselementes mit integrierten Gesichtsbräunern versehen ist. Das Wandelement und das Einstiegselement sind insbesondere schräg und spiegelbildlich zueinander angeordnet.

Jeweils mindestens ein Einstiegselement, zweckmäßigerweise ein an das Liegenelement vertikal anschließendes Einstiegselement, und ein zweites, oberes Einstiegselement sind einzeln oder gemeinsam verstellbar ausgebildet und können aus einer Betriebsstellung, in der sie spiegelbildlich zu einem oder auch zwei angeordneten Wandelementen gehalten sind, in eine Offenstellung bewegt werden, in der ein bequemer Einstieg in die Bräunungskammer möglich ist.

In einer besonders einfachen und raumsparenden Konstruktion können die Einstiegselemente in vertikaler Richtung am Gehäuse, beispielsweise in entsprechendën Führungsschienen, verschoben werden, bis die Einstiegsöffnung freigegeben ist. Es können auch Aufnahmeräume oder Ausnehmungen im beispielsweise schrankartigen Gehäuse für verstellte Einstiegselemente ausgebildet sein. Eine Verstellung der Einstiegselemente erfolgt zweckmäßigerweise, nachdem das obere Einstiegselement, das am unteren, vertikalen Einstiegselement angelenkt ist und das in Betriebsstellung schräg zwischen dem unteren Einstiegselement und dem Deckenelement verläuft, in eine vertikale, mit dem unteren Einstiegselement fluchtende Stellung verschwenkt wurde.

Zur Vergrößerung der Einstiegsöffnung empfiehlt es sich, auch das Deckenelement, das bevorzugt einteilig ausgebildet und in Betriebsstellung horizontal angeordnet ist, verstellbar zu befestigen. Wenn im angrenzenden Bereich des gemeinsamen Gehäuses eine Ausnehmung ausgebildet ist, kann das Deckenelement beispielsweise in einer aufgeschwenkten oder auch verschobenen Stellung besonders einfach und sicher während des Einstiegs gehalten werden.

Eine bevorzugte erfindungsgemäße Anordnung, die sich durch eine übersichtliche Konstruktion des Gehäuses und der Umkleideräume auszeichnet, weist zwei Ganzkörper-Bräunungsgeräte in einem schrankartigen Gehäuse doppelstöckig und miteinander fluchtend auf. Das Gehäuse kann in dieser Anordnung im Vertikalschnitt die Form eines stilisierten S aufweisen, in dessen konkaven S-Bogen-Seiten sich jeweils eine Einstiegsöffnung eines Ganzkörper-Bräunungsgerätes befindet. Die Ausnehmung für ein aufschwenkbares Deckenelement des unteren Ganzkörper-Bräunungsgerätes ist in oder an einem Zwischenboden des gemeinsamen Gehäuses ausgebildet. Die Ausnehmung für das Deckenelement des oberen Ganzkörper-Bräunungsgerätes befindet sich in oder an der Gehäusedecke.

Die Einstiegselemente des oberen Ganzkörper-Bräunungsgerätes können zur Freigabe eines Einstiegsbereiches in einer zweckmäßigen und platzsparenden Weise in einen Aufnahmeraum im Bereich der Gehäusedecke verschoben werden, während die Einstiegselemente des unteren Ganzkörper-Bräunungsgerätes in einen oberen Bereich der Außenseite der Gehäusewand verschiebbar sind.

Eine doppelstöckige und in verstärktem Maße eine mehrstöckige Anordnung von zwei oder mehreren Ganzkörper-Bräunungsgeräten, die über- und/oder nebeneinander oder auch in vertikaler Richtung versetzt angeordnet sein können, weisen neben einer effizienten Flächenausnutzung auch energetische Vorteile auf, da ein gemeinsames Kühlsystem, beispielsweise ein gemeinsamer Kühlluftschacht, und eine gemeinsame Abluftabsaugung installiert werden können.

Bei einer Doppelstockanordnung der Ganzkörper-Bräunungsgeräte können in einem bodenseitigen Sockel des gemeinsamen Gehäuses eine Ansaugöffnung und im Bereich der Gehäusedecke eine Abluftabsaugung vorgesehen sein. Selbstverständlich kann umgekehrt auch die Abluftabsaugung nahe dem Gehäuseboden installiert sein, während im deckenseitigen Bereich des gemeinsamen Gehäuses Frischluft einströmt bzw. angesaugt wird.

Es ist vorteilhaft, die Bräunungskammern der einzelnen Ganzkörper-Bräunungsgeräte an eine Zentralluftleitung anzuschließen, um trotz der Wärmeentwicklung der Ganzkörper-Bräunungsgeräte für eine gute Luft in den Bräunungskammern zu sorgen.

Konstruktions- und fertigungstechnische Vorteile ergeben sich, wenn der Kühlluftschacht bei zwei doppelstöckig angeordneten Ganzkörper-Bräunungsgeräten entlang dem im Vertikalschnitt S-förmigen Gehäuse verläuft.

Außerdem kann eine entsprechend ausgebildete Zugangsvorrichtung der erfindungsgemäßen Anordnung zu einer effektiven Flächennutzung beitragen. Wenn beispielsweise als Zugangsvorrichtung zum oberen Ganzkörper-Bräunungsgerät einer Doppelstockanordnung ein über eine Treppe zugängliches Podest vorgesehen ist, kann der Raum unterhalb des Podestes zur Unterbringung eines Klimagerätes aber auch anderer Geräte zum Betrieb der Ganzkörper-Bräunungsgeräte ausgenutzt werden.

In einer besonders bevorzugten Weiterbildung der Erfindung - ist vorgesehen, zwei-, drei- oder auch mehrstöckig in einem gemeinsamen Gehäuse angeordnete Ganzkörper-Bräunungsgeräte und zugehörige Umkleideräume oder Umkleidekabinen sowie Zugangsvorrichtungen als eine containerartige Einheit in einer zentralen Fertigungsstätte vorzufertigen und am jeweiligen Einsatzort lediglich aufzustellen und an die Versorgungsleitungen anzuschließen. Derartige containerartige Einheiten, die in beliebiger Anzahl und Anordnung aneinandergereiht oder auch übereinander angeordnet werden können, ermöglichen eine kostengünstige Fertigung und eine besonders schnelle Einrichtung eines Sonnenstudios.

Bei einer Doppelstockanordnung in einer derartigen containerartigen Einheit ist das gemeinsame Gehäuse mit einem unteren und einem oberen Ganzkörper-Bräunungsgerät zweckmäßigerweise zentral zwischen zwei an die Einstiegsöffnungen der Ganzkörper-Bräunungsgeräte angrenzenden Umkleideräumen angeordnet. Ein Abluftkanal für den gemeinsamen Kühlluftschacht ist vorteilhafterweise im Umkleideraum des unteren Ganzkörper-Bräunungsgerätes installiert. Im Umkleideraum für das obere Ganzkörper-Bräunungsgerät sind ein Podest und eine Treppe vorhanden, die einen bequemen Einstieg in die Bräunungskammer des oberen Ganzkörper-Bräunungsgerätes ermöglichen.

Prinzipiell können in einer weiteren Ausgestaltung auch die einzelnen Ganzkörper-Bräunungsgeräte oder eine Geräteeinheit aus mehreren Ganzkörper-Bräunungsgeräten verstellbar, beispielsweise in vertikaler oder horizontaler Richtung verschiebbar oder auf einer schiefen Ebene bewegbar sein. Es besteht dann die Möglichkeit, ein einzelnes Ganzkörper-Bräunungsgerät zum Ein- oder Ausstieg zu einem gemeinsamen Umkleideraum mit Einzelkabinen oder an einzeln zugeordnete Umkleideräume oder Umkleidekabinen zu bewegen.

Weiterführende Ausgestaltungen sind eine fahrstuhlartige Bewegung von mehreren übereinander angeordneten Ganzkörper-Bräunungsgeräten. Zweckmäßigerweise sollte hierbei eine Steuerung in Abhängigkeit von den eingestellten Bräunungszeiten die Bewegung der Geräteeinheit übernehmen, so daß ein ungestörter Ein- und Ausstieg aus einem Ganzkörper-Bräunungsgerät gewährleistet ist.

Bei über- und nebeneinander an einer gemeinsamen Fördervorrichtung befestigten Ganzkörper-Bräunungsgeräten kann eine paternosterartige Umlaufbewegung zur Überwindung der Höhendifferenz zwischen mindestens einem Fußboden und dem jeweiligen Ganzkörper-Bräunungsgerät dienen. Auch hier ist eine Steuerung der Umlaufbewegung sinnvoll. In Abhängigkeit von der Anzahl der Ganzkörper-Bräunungsgeräte in einer derartigen Einheit können an beiden Außenseiten der Geräteeinneit Umkleideräume oder Umkleidekabinen angeordnet sein.

Vorteilhaft ist schließlich auch eine kreisförmig bewegbare Doppelstockanordnung von mindestens zwei Ganzkörper-Bräunungsgeräten, der ein unterteilter Umkleideraum oder aber ein Umkleideraum mit Kabinen auf einer Einstiegsseite zugeordnet sein können. Die Stirnseiten von zwei Ganzkörper-Bräunungsgeräten sind hierbei jeweils an einem Ende eines drehbar gelagerten zweiarmigen Hebels angelenkt, wobei die Drehachse für den Hebel jeweils zwischen den Ganzkörper-Bräunungsgeräten und etwa in der Mitte des Hebels angeordnet ist.

Insbesondere bei den zuletzt beschriebenen bewegbaren Geräteeinheiten ist eine blickdichte Ausbildung eines Ganzkörper-Bräunungsgerätes von Vorteil, weil dadurch die Verkleidung eines gemeinsamen Gehäuses oder aber eine Abdeckung der Garderobenwände in einfacher Weise vorgenommen werden kann.

Darüber hinaus besteht die Möglichkeit, zur Überwindung von Höhendifferenzen auch Rolltreppen oder Rollbahnen, bewegbare Treppen, Steigleitern oder ähnliches zu verwenden.

Die Erfindung wird nachsehend anhand einer Zeichnung weiter erläutert; in dieser zeigen
- Fig. 1: einen Vertikalschnitt einer ersten Ausführungsform einer erfindungsgemäßen Anordnung zur Ganzkörper-Bräunung;
- Fig. 2: eine Draufsicht auf die Anordnung gemäß Fig. 1;
- Fig. 3: eine stark schematisierte Darstellung einer zweiten Ausführungsform einer erfindungsgemäßen Anordnung zur Ganzkörper-Bräunung mit drei übereinander angeordneten Ganzkörper-Bräunungsgeräten;
- Fig. 4: eine schematische Darstellung einer erfindungsgemäßen Anordnung mit mehr als drei übereinander angeordneten Ganzkörper-Bräunungsgeräten;
- Fig. 5: eine schematische Darstellung einer erfindungsgemäßen Anordnung mit drei übereinander angeordneten und fahrstuhlartig bewegbaren Ganzkörper-Bräunungsgeräten;
- Fig. 6: eine schematische Darstellung einer erfindungsgemäßen Anordnung mit acht über- und nebeneinander angeordneten und paternosterartig bewegbaren Ganzkörper-Bräunungsgeräten und
- Fig. 7: eine schematische Darstellung einer erfindungsgemäßen Anordnung mit zwei doppelstöckig angeordneten und kreisförmig bewegbaren Ganzkörper-Bräunungsgeräten.

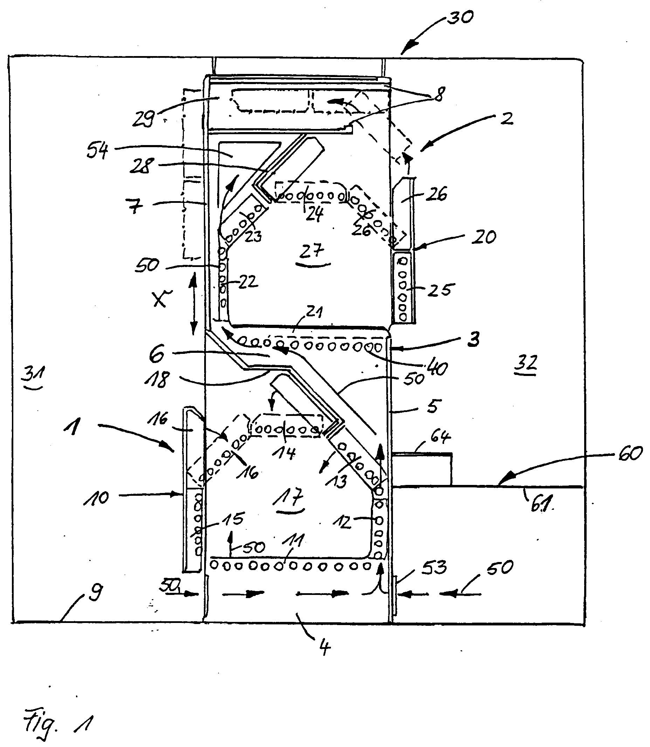

In Fig. 1 ist in einem schematischen Vertikalschnitt eine doppelstöckige Anordnung zur Ganzkörper-Bräunung dargestellt.

Ein unteres Ganzkörper-Bräunungsgerät 1 und ein oberes Ganzkörper-Bräunungsgerät 2 sind in einem gemeinsamen schrankartigen Gehäuse 3 übereinander angeordnet. Das untere Ganzkörper-Bräunungsgerät 1 weist ein horizontales Liegenelement 11 auf, welches muldenförmig, der Körperkontur entsprechend ausgebildet und auf einem bodenseitigen Sockel 4 des gemeinsamen Gehäuses befestigt ist. An einer längsseitigen Gehäusewand 5 sind, angrenzend an das Liegenelement 11, ein unteres, vertikales Wandelement 12 und ein oberes, schräg angeordnetes Wandelement 13 befestigt. Das untere Wandelement 12 ist fest angeordnet, während das obere Wandelement 13 neigungsverstellbar an der Gehäusewand 5 oder auch am unteren Wandelement 12 befestigt sein kann. Das obere Wandelement 13 reicht bis nahe an ein Deckenelement 14 heran, welches in Betriebsstellung horizontal und parallel zum Liegenelement 11 verläuft.

Zwischen dem Deckenelement 14 und dem Liegenelement 11 sind spiegelbildlich zu den Wandelementen 12, 13 ein unteres und ein oberes Einstiegselement 15, 16 vorgesehen, welche eine Einstiegsöffnung 10 im unteren Bereich einer Gehäusewand 7 verschließen können und welche wie die übrigen Elemente 11 bis 14 des unteren Ganzkörper-Bräunungsgerätes 1 mit UV-Strahlern 40 versehen sind.

Als UV-Strahler 40 sind hierbei in Längsrichtung verlaufende Röhren eingesetzt, die in einem regelmäßigen Abstand nebeneinander in den einzelen Elementen 11 bis 16 des Ganzkörper-Bräunungsgerätes 1 angeordnet sind. Aufgrund der Anordnung dieser Elemente 11 bis 16 in einer Betriebsstellung ist eine blickdicht abgeschlossene Bräunungskammer 17 gebildet, die eine allseitige gleichmäßige Ganzkörper-Bräunung gewährleistet.

Eine haubenartige Anordnung aus einem höhenverstellbaren Deckenelement 14 und einem neigungsverstellbaren, schrägen oberen Einstiegs- und Wandelement 16, 13 ist besonders vorteilhaft geeignet, individuelle Bräunungswünsche zu realisieren.

Ein Einstieg in die untere Bräunungskammer 17 erfolgt nach einer Verstellung der Einstiegselemente 15, 16 in eine Öffnungsstellung, in der die Einstiegsöffnung 10 freigegeben ist. In diesem Ausführungsbeispiel werden die Einstiegselemente 15, 16 an der Außenseite der Gehäusewand 7 in schienenartigen Führungen (nicht dargestellt) bewegt, nachdem das obere Einstiegselement 16, welches am unteren Einstiegselement 15 angelenkt ist, in eine vertikale Stellung geschwenkt wurde (siehe Doppelpfeil X). In Fig. 1 ist diese vertikale Stellung der Einstiegselemente 15, 16, die gegebenenfalls durch Rast- oder Feststellelemente gesichert sein kann, mit durchgezogenen Linien dargestellt. Die Betriebsstellung des oberen Einstiegselementes 16 ist mit gestrichelten Linien und die Öffnungsstellung im oberen Bereich der Gehäusewand 7 ist mit strichpunktierten Konturen kenntlich gemacht.

Eine vertikale Verstellung der Einstiegselemente 15, 16 ist besonders vorteilhaft, wenn diese Einstiegselemente 15, 16 analog zu den übrigen Elementen 11 bis 14 des unteren Ganzkörper-Bräunungsgerätes 1 in Längsrichtung einteilig ausgebildet sind.

Wenn die Einstiegselemente 15, 16 in Form von schenkbar aneinander angelenkten Segmenten angeordnet sind, könnte eine horizontale Verstellung in einen Fußbereich in der Art einer Falttür erfolgen. Möglich wäre auch ein Verschwenken der Segmente in einen angrenzenden Umkleideraum 31.

Um einen unbehinderten, bequemen Einstieg in das Ganzkörper-Bräunungsgerät 1 zu ermöglichen, ist auch das Dekkenelement 14 aus seiner horizontalen Betriebsstellung (gestrichelte Linien) in eine Öffnungsstellung (durchzogene Linien) schwenkbar. Eine gesicherte Halterung des Deckenelementes 14 in der Öffnungsstellung erfolgt in einer Ausnehmung 18, die an einem Zwischenboden 6 des gemeinsamen Gehäuses 3 ausgebildet ist und gegebenenfalls Halteelemente aufweisen kann.

Der Zwischenboden 6 verläuft nahezu horizontal über die gesamte Länge zwischen den zueinander parallelen Gehäusewänden 5, 7 des gemeinsamen Gehäuses 3 und trennt das erste untere Ganzkörper-Bräunungsgerät 1 von dem zweiten, oberen Ganzkörper-Bräunungsgerät 2. Dieses obere Ganzkörper-Bräunungsgerät 2 ist grundsätzlich wie das untere Ganzkörper-Bräunungsgerät 1 ausgebildet und weist somit ebenfalls ein Liegenelement 21, zwei Wandelemente 22, 23, ein oberes, aufschwenkbares Deckenelement 24 und zwei Einstiegselemente 25, 26 auf, die mit UV-Strahlern versehen sind und eine zweite Bräunungskammer 27 umschließen.

In einer vorteilhaften Weise sind die vertikalen bzw. schräg angeordneten Wand- und Einstiegselemente 22, 23 bzw. 25, 26 jedoch im Vergleich zum unteren Ganzkörper-Bräunungsgerät 1 seitenverkehrt angeordnet, so daß die Einstiegsöffnungen 10, 20 der übereinander angeordneten Ganzkörper-Bräunungsgeräte 1, 2 an einander gegenüberliegenden Wandbereichen der Gehäusewände 5, 7, nämlich in den konkaven S-Bogen des im Vertikalschnitt S-förmigen gemeinsamen Gehäuses 3 angeordnet sind. Eine Verstellung der Einstiegselemente 25, 26 des oberen Ganzkörper-Bräunungsgerätes 2 erfolgt ähnlich wie beim unteren Ganzkörper-Bräunungsgerät 1, indem das obere Einstiegselement 26 aus einer schrägen Betriebsstellung (gestrichelte Linien) in eine vertikale Stellung (durchzogene Linien) geschwenkt und am unteren Einstiegselement 25 in dieser Stellung gesichert wird. Ähnlich einer Jalousie können die Einstiegselemente 25, 26 dann in einem Aufnahmeraum 29 untergebracht werden, der horizontal in einer oberen Gehäusedecke 8 des gemeinsamen Gehäuses 3 ausgebildet ist. Die Verstellung der Einstiegselemente 15, 16 bzw. 25, 26, eine gegebenenfalls ausgebildete Höhenverstellung der Deckenelemente 14 bw. 24 und die Benutzung von in den Deckenelementen angeordneten Gesichtsbräunern kann vorteilhafterweise mittels Fernbedienung erfolgen.

Die Gehäusewände 5, 7 sind im Bereich der Wandelemente 12, 13 bzw. 22, 23 mit einer großflächigen und blickdichten Verkleidung versehen (nicht dargestellt). Im Bereich der Einstiegsöffnungen 10, 20 sind auf die Randbereiche, beispielsweise den Fußbereich, beschränkte Verkleidungsflächen angebracht. Auf diese Weise ist eine Einsicht in die Bräunungskammern 17, 27 nur von einem zugehörigen, angrenzenden Umkleideraum 31, 32 aus möglich und auch nur in Öffnungsstellung der jeweiligen Einstiegselemente 15, 16 bzw. 25, 26.

Da die UV-Strahler 40 neben der zur Bräunung benötigten elektromagnetischen Energie auch Wärmeenergie erzeugen, ist eine Kühlung der Strahler bzw. der Bräunungskammern 17, 27 vorgesehen. Zu diesem Zweck wird, wie in Fig. 1 gezeigt, entlang eines gemeinsamen Kühlluftschachtes, der durch Pfeile 50 schematisch angedeutet ist und entlang dem S-förmigen Vertikalschnitt des gemeinsamen Gehäuses 3 verläuft, Kühlluft transportiert und gegebenenfalls umgewälzt. Die Kühlluft wird über eine Ansaugöffnung 53 im bodenseitigen Sockel 4 in das gemeinsame Gehäuse 3 durch ein nicht dargestelltes Gebläse angesaugt und zu den entsprechenden Strahlern 40 der Ganzkörper-Bräunungsgeräte 1, 2 transportiert. Sinnvoll ist eine Abkühlung der angesaugten Luft in einem Klimaaggregat (nicht dargestellt), das in einem Podest 61 angeordnet werden kann.

Das Podest 61, welches als Zugangsvorrichtung 60 zur Überwindung der Höhendifferenz zwischen einem Fußboden 9 in Normalhöhe, wie er im Umkleideraum 31 des unteren Ganzkörper-Bräunungsgerätes 1 vorliegt, und dem oberen Ganzkörper-Bräunungsgerät 2 ermöglicht den Zugang zur Bräunungskammer 27.

Im Bereich der Gehäusedecke 8 ist eine Abluftabsaugung 54 vorgesehen. Die Strömungsrichtung der Kühlluft kann auch in der entgegengesetzten Richtung erfolgen.

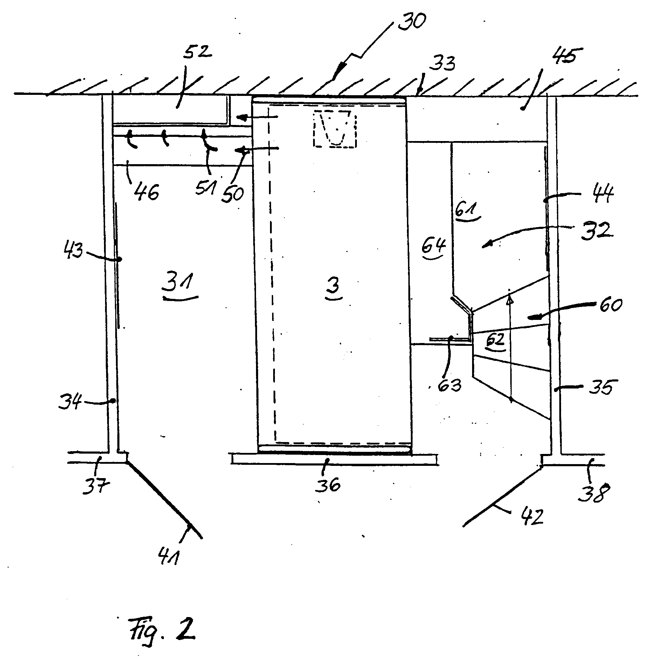

In Fig. 2 ist eine vorgefertigte containerartige Einheit 30 mit einer doppelstöckigen Anordnung gemäß Fig. 1 in einer stark schematisierten Draufsicht dargestellt. Gleiche Merkmale sind mit identischen Bezugszeichen versehen.

Die containerartige Einheit 30 umfaßt in dem dargestellten Ausführungsbeispiel eine Rückwand 33, zwei Seitenwände 34, 35, Frontwandsegmente 36, 37 und 38, zwischen denen eine verschließbare Tür 41 für den Umkleideraum 31 des unteren Ganzkörper-Bräunungsgerätes 1 und eine verschließbare Tür 42 für den Umkleideraum 32 des oberen Ganzkörper-Bräunungsgerätes 2 angeordnet sind. Im Ausführungsbeispiel sind die Türen 41, 42 als Schwenktüren ausgebildet. Bevorzugt sind jedoch platzsparende Falttüren oder auch als Rolläden ausgebildete Türen. Die Umleidekabine 31 des unteren Ganzkörper-Bräunungsgerätes 1 ist mit einem Fußboden 9 in Normalhöhe versehen und weist im rückwandseitigen Bereich einen Abluftkanal 52 auf, der mit der Abluftsaugung 54 des ge - meinsamen Gehäuses 3 verbunden werden kann. Der Abluftkanal 52 ist zweckmäßigerweise durch eine Garderobe 46 verkleidet. An der Seitenwand 34 ist ein Spiegel 43 angeordnet.

Dem Ganzkörper-Bräunungsgerät 2 ist die Umkleidekabine 32 zugeordnet. Diese Umkleidekabine 32 ist grundsätzlich ebenso groß wie die Umkleidekabine 31, weist aber das im Zusammenhang mit der Fig. 1 beschriebene Podest 61 und eine Treppe 62 zwischen der Zugangstür 42 und dem Podest 61 als Zugangsvorrichtung 60 auf. Die Treppe 62 ist mit einem Geländer oder mit einem Handlauf 63 versehen. Im rückwandseitigen Teil des Umkleideraumes 32 ist eine Garderobe 45 angeordnet. Zwischen dem Podest 61 und dem gemeinsamen Gehäuse 3 ist ein schmaler, erhöhter Gang 64 belassen, um den Einstieg in die Bräunungskammer 27 zu erleichtern und um die Garderobe 45 zugänglich zu halten. Außerdem ist im Bereich des Podestes 61 an der Seitenwand 35 ein Spiegel 44 angebracht.

Die containerartige Einheit 30 mit einer doppelstöckigen Anordnung zweier Ganzkörper-Bräunungsgeräte 1, 2 erlaubt nicht nur eine herstellerseitige Vormontage der Ganzkörper-Bräunungsgeräte 1, 2 im gemeinsamen Gehäuse 3, sondern auch der jeweils zugehörigen Umkleideräume 31, 32. Vor Ort muß dann lediglich die Installation der elektrischen Ahschlüsse durchgeführt werden.

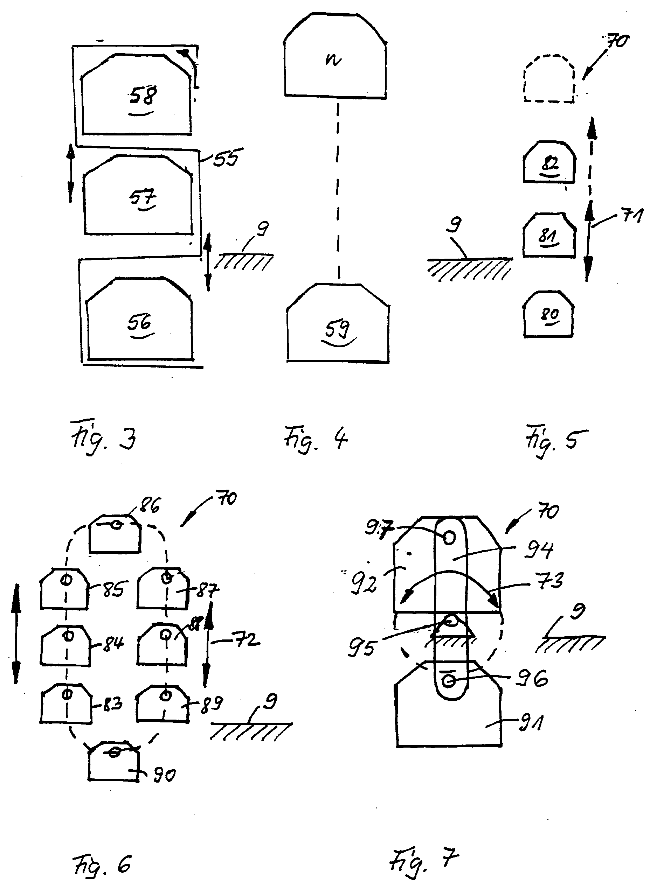

In Fig. 3 ist als ein zweites Ausführungsbeispiel der erfindungsgemäßen Anordnung zur Ganzkörperbräunung ein Schema einer dreistöckigen Anordnung mit einem in Fußbodenhöhe 9 zugänglichen ersten Ganzkörper-Bräunungsgerät 57, einem darunter angeordneten zweiten Ganzkörper-Bräunungsgerät 56 und einem über dem ersten Ganzkörper-Bräunungsgerät 57 angeordneten dritten Ganzkörper-Bräunungsgerät 58 wiedergegeben. Die drei Ganskörper-Bräunungsgeräte 56, 57, 58 sind in einem im Vertikalschhitt mäaanderförmigen gemeinsamen Gehäuse 55 angeordnet und jeweils über separate Einstiegsöffnungen (nicht dargestellt) zugänglich. Als Zugangsvorrichtungen (nicht dargestellt) für das obere Ganzkörper-Bräunungsgerät 58 und das untere Ganzkörper-Bräungsgerät 56 eignen sich neben Treppen und Podesten auch Rolltreppen. Die seitlich dargestellten Pfeile deuten die Verstellbewegungen der nicht dargestellten Einstiegselemente der drei Ganzkörper-Bräunungsgeräte 56, 57, 58 an.

Fig. 4 verdeutlicht eine mögliche Anordnung von mehr als drei übereinander Ganzkörper-Bräunungsgeräten 59 ... n. Der Zugang zu diesen Ganzkörper-Bräunungsgeräten wird durch eine Bewegung der Geräte selbst ermöglicht (nicht dargestellt). So ist es beispielsweise möglich, jedes einzelne Ganzkörper-Bräunungsgerät 59 ... n nach Bedarf von einem Umkleideraum, der mehrere Umkleidekabinen für einzelne Benutzer aufweist, zu benutzen.

Fig. 5 zeigt drei übereinander angeordnete Ganzkörper-Bräunungsgeräte 80, 81, 82, die zu einer Geräteeinheit 70 verbunden sind und zum Ein- und Ausstieg fahrstuhlartig (Pfeil 71) bewegt werden. Die Anordnung erlaubt einen ein- oder auch beidseitigen Ein- und Ausstieg in Höhe eine Fußbodens 9. Die gemeinsam bewegten Ganzkörper-Bräunungsgeräte 80, 81, 82 werden mit Hilfe eines Programmes mit der Eingabe der jeweiligen Bräunungszeit gesteuert.

Fig. 6 zeigt eine Geräteeinheit 70 mit 8 Ganzkörper-Bräunungsgeräten 83 bis 90, die einer paternosterartigen Bewegung unterworfen werden und schwenkbar an einer Bewegungsvorrichtung, welche mit gestrichelten Linien dargestellt ist, angeordnet sind. Die Ganzkörper-Bräunungsgeräte 83 bis 90 sind über- und nebeneinander sowie versetzt angeordnet und mit separat zugänglichen Einstiegsöffnungen (nicht dargestellt) versehen.

Fig. 7 zeigt eine doppelstöckige Anordnung zweier Ganzkörper-Bräunungsgeräte 91, 92, die kreisförmig an einem Hebel 94 bewegbar sind. Der Hebel 94 weist eine Drehachse 95 auf, die in der Mitte zwischen den Drehachsen 96, 97 der Ganzkörper-Bräunungsgeräte 91, 92 angeordnet ist. Die Geräteeinheit 70 ist so angeordnet, daß jeweils das obere Ganzkörper-Bräunungsgerät von einem Fußboden 9 aus zugänglich ist. Das gemeinsame Gehäuse (nicht dargestellt) kann aus einer Trägeranordnung mit einer lediglich den Einstiegsbereich begrenzenden Verkleidung bestehen.

## Patentansprüche

1. Anordnung zur Ganzkörper-Bräunung,
dadurch **gekennzeichnet,**
daß
a) wenigstens zwei Ganzkörper-Bräunungsgeräte (1, 2, 56-59, 80-92) mehrstöckig in einem gemeinsamen Gehäuse (3, 55) angeordnet sind,
b) daß jedes Ganzkörper-Bräunungsgerät (1,2, 56-59, 80-92) mehrere mit UV-Strahlern (4) versehene Elemente (11-16; 21-26) aufweist, welche in ihrer Betriebsstellung jeweils eine Bräunungskammer (17, 27) umschließen,
c) daß die Ganzkörper-Bräunungsgeräte (1, 2, 56-59, 80-92) separate Einstiegsöffnungen (10, 20) aufweisen, welche durch mindestens ein Einstiegselement (15, 16; 25, 26) verschließbar sind,
d) daß ein erstes Ganzkörper-Bräunungsgerät (1) von einem Fußboden (9) aus zugänglich ist und
e) daß zur Überwindung einer Höhendifferenz zwischen dem Fußboden (9) und jedem weiteren Ganzkörper-Bräununggerät (2, 56-59, 80-92) eine Zugangsvorrichtung (60) vorgesehen ist.

2. Anordnung nach Anspruch 1,
dadurch **gekennzeichnet,**
daß die Einstiegsöffnungen (10; 20) der wenigstens zwei Ganzkörper-Bräunungsgeräte (1, 2) an einander gegenüberliegenden Seitenwänden (7, 5) des Gehäuses (3, 55) vorgesehen sind.

3. Anordnung nach Anspruch 1 oder 2,
dadurch **gekennzeichnet,**
daß jedes Ganzkörper-Bräunungsgerät (1, 2, 56-59, 80-92) ein Liegenelement (11; 21) und mindestens ein Wandelement (12, 13; 22, 23) sowie ein parallel zum Liegenelement (11; 21) angeordnetes Deckenelement (14; 24) aufweist und daß das mindestens eine Einstiegselement (15, 16; 25, 26) zwischen dem Liegenelement (11; 21) und dem Deckenelement (14; 24) angeordnet ist.

4. Anordnung nach einem der Ansprüche 1 bis 3,
dadurch **gekennzeichnet,**
daß angrenzend an das Liegenelement (11; 21) ein unteres, vertikales Einstiegselement (15; 25) und ein oberes Einstiegselement (16; 26) vorgesehen sind, welche am gemeinsamen Gehäuse (3, 55) zwischen einer Betriebsstellung und einer Öffnungsstellung verstellbar angeordnet sind.

5. Anordnung nach Anspruch 4,
dadurch **gekennzeichnet,**
daß das obere Einstiegselement (16; 26), das am unteren Einstiegselement (15; 25) schwenkbar gelagert ist, in Betriebsstellung schräg zwischen dem unteren Einstiegselement (15; 25) und dem Deckenelement (14; 24) gehalten ist und
daß zur Öffnungsstellung das in eine vertikale Stellung verschwenkte, obere Einstiegselement (16; 26) zusammen mit dem unteren Einstiegselement (15; 25) in vertikaler Richtung am Gehäuse (3, 55) verschiebbar ist.

6. Anordnung nach einem der Ansprüche 3 bis 5,
dadurch **gekennzeichnet,**
daß das Deckenelement (14, 24) zwischen einer horizontalen Betriebsstellung und einer Öffnungsstellung verstellbar angeordnet ist.

7. Anordnung nach einem der Ansprüche 3 bis 6,
dadurch **gekennzeichnet,**
daß spiegelbildlich zu den Einstiegselementen (15, 16; 25, 26) in Betriebsstellung an der jeweils gegenüberliegenden Gehäusewand (5,7) ein unteres, vertikales Wandelement (12; 22) und ein oberes, schräg angeordnetes Wandelement (13; 23) vorgesehen sind und
daß das Deckenelement (14; 24) mit dem schrägen Wandelement (13; 23) und dem schrägen Einstiegselement (16; 26) eine teilweise aufschwenkbare haubenartige Anordnung bildet.

8. Anordnung nach einem der vorhergehenden Ansprüche,
dadurch **gekennzeichnet,**
daß das gemeinsame Gehäuse (3) für ein doppelstöckig angeordnetes unteres und oberes Ganzkörper-Bräunungsgerät (1; 2) schrankartig ausgebildet ist, das im Vertikalschnitt die Form eines stilisierten S aufweist, und daß die Einstiegsöffnungen (10; 20) jeweils auf der konkaven Seite der S-Bogen angeordnet sind.

9. Anordnung nach Anspruch 8,
dadurch **gekennzeichnet,**
daß das gemeinsame Gehäuse (3) einen bodenseitigen Sockel (4), einen Zwischenboden (6) zwischen dem unteren und oberen Ganzkörper-Bräunungsgerät (1; 2) und eine Gehäusedecke (8) aufweist, daß die Wandelemente (12, 13) des unteren Ganzkörper-Bräunungsgerätes (1) an der Gehäusewand (5) und die Wandelemente (22, 23) des oberen Ganzkörper-Bräunungsgerätes (2) an der Gehäusewand (7) fixierbar sind.

10. Anordnung nach Anspruch 8 oder 9,
dadurch **gekennzeichnet,**
daß die Einstiegselemente (15, 16) des unteren Ganzkörper-Bräunungsgerätes (1) zur Öffnungsstellung bis zur Gehäusedecke (8) an der Außenseite der Gehäusewand (7) und die Einstiegselemente (25, 26) des oberen Ganzkörper-Bräunungsgerätes (2) in einen Aufnahmeraum (29) der Gehäusedecke (8) verschiebbar sind.

11. Anordnung nach einem der Ansprüche 8 bis 10,
dadurch **gekennzeichnet,**
daß am Zwischenboden (6) und an der Gehäusedecke (8) eine Ausnehmung (18; 28) zur Aufnahme des jeweils aufgeschwenkten Deckenelementes (14; 24) ausgebildet ist.

12. Anordnung nach einem der vorhergehenden Ansprüche
dadurch **gekennzeichnet,**
daß für die Ganzkörper-Bräunungsgeräte (1; 2, 56-59, 80-92) ein gemeinsamer Kühlluftschacht (50) vorgesehen ist, welcher die Bräunungskammern (17; 27) mit Kühlluft versorgt.

13. Anordnung nach Anspruch 12,
dadurch **gekennzeichnet,**
daß für die Kühlluft eine bodenseitige Ansaugöffnung (53) und eine deckenseitige Abluftabsaugung (54) im gemeinsamen Gehäuse (3, 55) vorgesehen sind.

14. Anordnung nach einem der Ansprüche 12 oder 13,
dadurch **gekennzeichnet,**
daß der gemeinsame Kühlluftschacht (50) bei zwei doppelstöckig angeordneten Ganzkörper-Bräunungsgeräten (1, 2) entlang dem S-förmigen Vertikalschnitt des gemeinsamen Gehäuses (3) verläuft.

15. Anordnung nach einem der Ansprüche 2 bis 14,
dadurch **gekennzeichnet,**
daß angrenzend an die Gehäusewand (7) und die Einstiegsöffnung (10) ein Umkleideraum (31) für das untere Ganzkörper-Bräunungsgerät (1) und angrenzend an die Gehäusewand (5) und die Einstiegsöffnung (20) ein Umkleideraum (32) für das obere Ganzkörper-Bräunungsgerät (2) angeordnet sind und
daß als Zugangsvorrichtung (60) ein über eine Treppe (62) zugängliches Podest (61) vorgesehen ist.

16. Anordnung nach Anspruch 15,
dadurch **gekennzeichnet,**
daß in dem Podest (61) Aggregate für den Betrieb der Ganzkörper-Bräunungsgeräte (1, 2), wie Klimageräte und Vorschaltgeräte, angeordnet sind.

17. Anordnung nach einem der Ansprüche 15 oder 16,
dadurch **gekennzeichnet,**
daß das gemeinsame Gehäuse (3) mit den mehrstöckig angeordneten Ganzkörper-Bräunungsgeräten (1, 2), den Umkleideräumen (31, 32) und der Zugangsvorrichtung (60) als containerartige Einheit (30) vorgefertigt ist.

18. Anordnung nach Anspruch 17,
dadurch **gekennzeichnet,**
daß die containerartige Einheit (30) einen Abluftkanal (52) aufweist und daß jeder Umkleideraum (31; 32) über eine separate Tür (41; 42) zugänglich ist.

19. Anordnung nach Anspruch 18,
dadurch **gekennzeichnet,**
daß der Abluftkanal (52) im Umkleideraum (31) für das untere Ganzkörper-Bräunungsgerät (1) angeordnet ist,
daß jeder Umkleideraum (31, 32) mit einer Garderobe (45; 46) und einem Spiegel (43; 44) versehen ist und daß die Türen (41; 42) der Umkleideräume (31; 32) als platzsparende Rolltüren ausgebildet sind.

20. Anordnung nach einem der vorhergehenden Ansprüche 1 bis 7, 12, 13,
dadurch **gekennzeichnet,**
daß in dem gemeinsamen Gehäuse (55), das im Vertikalschnitt mäanderförmig ausgebildet ist, oberhalb von zwei Ganzkörper-Bräunungsgeräten (56, 57) ein drittes Ganzkörper-Bräunungsgerät (58) angeordnet ist, welches über eine weitere Zugangsvorrichtung und einen separaten Umkleideraum zugänglich ist.

21. Anordnung nach einem der vorhergehenden Ansprüche 1 bis 7, 12, 13,
dadurch **gekennzeichnet,**
daß mehrere Ganzkörper-Bräunungsgeräte (59...n) übereinander oder in vertikaler Richtung versetzt angeordnet sind und daß die Zugangsvorrichtung und/oder die Ganzkörper-Bräunungsgeräte (59...n) verstellbar sind.

22. Anordnung nach einem der vorhergehenden Ansprüche 1 bis 7, 12, 13,
dadurch **gekennzeichnet,**
daß mehrere Ganzkörper-Bräunungsgeräte (80, 81, 82; 83-90; 91, 92) zu einer Geräteeinheit (70) angeordnet sind und daß zur Überwindung der Höhendifferenz zwischen einem Fußboden und den Einstiegsöffnungen die gesamte Geräteeinheit (70) und/oder die einzelnen Ganzkörper-Bräunungsgeräte (80, 81, 82; 83-90; 91, 92) bewegbar sind.

23. Anordnung nach einem der vorhergehenden Ansprüche 1 bis 7,
dadurch **gekennzeichnet,**
daß mehrere Ganzkörper-Bräunungsgeräte (80, 81, 82; 83-90; 91, 92) zu einer Geräteeinheit (70) angeordnet sind und daß zur Überwindung der Höhendifferenz zwischen einem Fußboden und den Einstiegsöffnungen die gesamte Geräteeinheit (70) und/oder die einzelnen Ganzkörper-Bräunungsgeräte (80, 81, 82; 83-90; 91, 92) bewegbar sind.

24. Anordnung nach Anspruch 23,
dadurch **gekennzeichnet,**
daß die Geräteeinheit (70) übereinander angeordnete Ganzkörper-Bräunungsgeräte (80, 81, 82) aufweist, die fahrstuhlartig bewegbar sind.

25. Anordnung nach Anspruch 23,
dadurch **gekennzeichnet,**
daß die Geräteeinheit (70) übereinander und nebeneinander angeordnete Ganzkörper-Bräunungsgeräte (83-90) aufweist, die paternosterartig bewegbar sind.

26. Anordnung nach Anspruch 23,
dadurch **gekennzeichnet,**
daß in der Geräteeinheit (70) zwei Ganzkörper-Bräunungsgeräte (91, 92), die in Betriebsstellung übereinander angeordnet sind, kreisförmig bewegbar sind.

27. Anordnung nach Anspruch 26,
dadurch **gekennzeichnet,**
daß die Ganzkörper-Bräunungsgeräte (91, 92) um ihre Längsachsen (96, 97) drehbar an einem Hebel (94) gelagert sind und daß eine Drehachse (95) des Hebels (94) mittig zwischen den zwei Ganzkörper-Bräunungsgeräten (91, 92) angeordnet ist.

## Claims

1. Whole body tanning arrangement,
**characterized** in that
a) at least two whole body tanning devices (1, 2, 56-59, 80-92) are arranged in multistorey manner in a common housing (3, 55),
b) each whole body tanning device (1, 2, 56-59, 80-92) has several elements (11-16; 21-26) provided with UV-lamps (4) and which in their operating position in each case surround a tanning chamber (17, 27),
c) the whole body tanning devices (1, 2, 56-59, 80-92) have separate entrance openings (10, 20), which can be closed by at least one entrance element (15, 16; 25, 26),
d) a first whole body tanning device (1) is accessible from a floor (9) and
e) for overcoming a height difference between the floor (9) and each further whole body tanning device (2, 56-59, 80-92) an access device (60) is provided.

2. Arrangement according to claim 1,
**characterized** in that
the entrance openings (10, 20) of the at least two whole body tanning devices (1, 2) are provided on opposite side walls (7, 5) of the housing (3, 55).

3. Arrangement according to claim 1 or 2,
**characterized** in that
each whole body tanning device (1, 2, 56-59, 80-92) has a lying element (11; 21) and at least one wall element (12, 13; 22, 23), as well as a ceiling element (14; 24) parallel to the lying element (11; 21) and that the at least one entrance element (15, 16; 25, 26) is located between the lying element (11; 21) and the ceiling element (14; 24).

4. Arrangement according to one of the claims 1 to 3,
**characterized** in that
adjacent to the lying element (11; 21) are provided a lower, vertical entrance element (15; 25) and an upper entrance element (16; 26), which are adjustably located between an operating position and an opening position at the common housing (3; 55).

5. Arrangement according to claim 4,
**characterized** in that
the upper entrance element (16; 26), which is pivotably mounted on the lower entrance element (15; 25) is held in the operating position in sloping manner between the lower entrance element (15; 25) and the ceiling element (14; 24) and for the opening position the upper entrance element (16; 26) pivoted into a vertical position, together with the lower entrance element (15, 25), can be vertically displaced at the housing (3, 55).

6. Arrangement according to one of the claims 3 to 5,
**characterized** in that
the ceiling element (14, 24) is adjustably arranged between a horizontal operating position and an opening position.

7. Arrangement according to one of the claims 3 to 6,
**characterized** in that
in mirror image to the entrance elements (15, 16; 25, 26) in the operating position on the in each case opposite housing wall (5, 7) are provided a lower, vertical wall element (12, 22) and an upper, sloping wall element (13; 23) and that the ceiling element (14; 24) forms a partly upwardly pivotable, hood-like arrangement together with the sloping wall element (13; 23) and the sloping entrance element (16; 26).

8. Arrangement according to one of the preceding claims,
**characterized** in that
the common housing (3) for a lower and upper whole body tanning device (1; 2) arranged in two-storey manner has a cupboard-like construction which, in vertical section is in the form of a stylized S and that the entrance openings (10; 20) are in each case located on the concave side of the S-curve.

9. Arrangement according to claim 8,
**characterized** in that
the common housing (3) has a bottom-side base (4), a false floor (6) between the lower and upper whole body tanning device (1, 2) and a housing ceiling (8) and that the wall elements (12; 13) of the lower whole body tanning device (1) can be fixed to the housing wall (5) and the wall elements (22, 23) of the upper whole body tanning device (2) to the housing wall (7).

10. Arrangement according to claim 8 or 9,
**characterized** in that
the entrance elements (15, 16) of the lower whole body tanning device (1) for the opening position can be displaced up to the housing ceiling (8) at the outside of the housing wall (7) and the entrance elements (25, 26) of the upper whole body tanning device (2) into a reception area (29) of the housing ceiling (8).

11. Arrangement according to one of the claims 8 to 10,
**characterized** in that
a recess (18; 28) for receiving the in each case upwardly pivoted ceiling element (14; 24) is formed on the false floor (6) and on the housing ceiling (8).

12. Arrangement according to one of the preceding claims,
**characterized** in that
a common cooling air shaft (50) supplying cooling air to the tanning chambers (17; 27) is provided for the whole body tanning devices (1, 2, 56-59, 80-92).

13. Arrangement according to claim 12,
**characterized** in that
for the cooling air there is a bottom-side intake (53) and a ceiling-side used air extractor (54) in the common housing (3, 55).

14. Arrangement according to one of the claims 12 or 13,
**characterized** in that
the common cooling air shaft (50) in the case of two whole body tanning devices (1, 2) arranged in two-storey manner passes along the S-shaped vertical section of the common housing (3).

15. Arrangement according to one of the claims 2 to 14,
**characterized** in that
adjacent to the housing wall (7) and the entrance opening (10) are provided a changing room (31) for the lower whole body tanning device (1) and adjacent to the housing wall (5) and entrance opening (20) a changing room (32) for the upper whole body tanning device (2) and that the access means (60) is constituted by a platform (61) accessible by means of steps (62).

16. Arrangement according to claim 15,
**characterized** in that
the platform (61) contains units for operating the whole body tanning devices (1, 2) such as air conditioning devices and power supply units.

17. Arrangement according to one of the claims 15 or 16,
**characterized** in that
the common housing (3) is prefabricated as a container-like unit (30) together with the whole body tanning devices (1, 2) arranged in multistorey manner, the changing rooms (31, 32) and the access means (60).

18. Arrangement according to claim 17,
**characterized** in that
the container-like unit (30) has a used air duct (52) and that each changing room (31; 32) is accessible by means of a separate door (41; 42).

19. Arrangement according to claim 18,
**characterized** in that
the used air duct (52) is located in the changing room (31) for the lower whole body tanning device (1), that each changing room (31, 32) is provided with a wardrobe (45; 46) and a mirror (43; 44) and that the doors (41; 42) of the changing rooms (31; 32) are constructed as space-saving rolling doors.

20. Arrangement according to one of the preceding claims 1 to 7, 12 and 13,
**characterized** in that
in the common housing (55), which is meander-shaped in vertical section, above two whole body tanning devices (56, 57) is provided a third whole body tanning device (58), which is accessible by means of a further access means and a separate changing room.

21. Arrangement according to one of the preceding claims 1 to 7, 12 and 13,
**characterized** in that
several whole body tanning devices (59 ... n) are arranged in superimposed or vertically displaced manner and that the access means and/or whole body tanning devices (59 ... n) are adjustable.

22. Arrangement according to one of the preceding claims 1 to 7, 12 and 13,
**characterized** in that
several whole body tanning devices (80, 81, 82; 83-90; 91, 92) are arranged as an equipment unit (70) and that for overcoming the height difference between a floor and the entrance openings the entire equipment unit (70) and/or the individual whole body tanning devices (80, 81, 82, 83-90; 91, 92) are movable.

23. Arrangement according to one of the preceding claims 1 to 7,
**characterized** in that
several whole body tanning devices (80, 81, 82; 83-90; 91, 92) are formed into an equipment unit (70) and that for overcoming the height difference between a floor and the entrance openings the entire equipment unit (70) and/or the individual whole body tanning devices (80, 81, 82; 83-90; 91, 92) are movable.

24. Arrangement according to claim 23,
**characterized** in that
the equipment unit (70) has superimposed whole body tanning devices (80, 81, 82), which can be moved in lift-like manner.

25. Arrangement according to claim 23,
**characterized** in that
the equipment unit (70) has superimposed and juxtaposed whole body tanning devices (83-90), which can be moved in paternoster-like manner.

26. Arrangement according to claim 23,
**characterized** in that
in the equipment unit (70) two whole body tanning devices (91, 92), which are superimposed in the operating position, are movable in circular manner.

27. Arrangement according to claim 26,
**characterized** in that
the whole body tanning devices (91, 92) are mounted on a lever (94) so as to be rotatable about the longitudinal axes (96, 97) thereof and that a rotation axis (95) of the lever (94) is positioned centrally between the two whole body tanning devices (91, 92).

## Revendications

1. Ensemble de bronzage intégral, ***caractérisé en ce que***
a) au moins deux appareils de bronzage intégral (1, 2, 56-59, 80-92) sont disposés sur plusieurs étages dans un logement commun (3, 55),
b) ***en ce que*** chaque appareil de bronzage intégral (1, 2, 56-59, 80-92) présente plusieurs éléments (11-16 ; 21-26) munis de projecteurs à UV (40), éléments qui, dans leur position de service, entourent chacun une chambre de bronzage (17, 27),
c) ***en ce que*** les appareils de bronzage intégral (1, 2, 56-59, 80-92) présentent des ouvertures d'entrée séparées (10, 20) qui peuvent être fermées par au moins un élément d'entrée (15, 16 ; 25, 26),
d) ***en ce qu'***un premier appareil de bronzage intégral (1) est accessible depuis un plancher (9), et
e) ***en ce que*** pour surmonter une différence de hauteur entre le plancher (9) et chaque autre appareil de bronzage intégral (2, 56-59, 80-92), il est prévu un dispositif d'accès (60).

2. Ensemble selon la Revendication 1, ***caractérisé en ce que*** les ouvertures d'entrée (10 ; 20) desdits au moins deux appareils de bronzage intégral (1, 2) sont prévues sur des parois latérales mutuellement opposées (7, 5) du logement (3, 55).

3. Ensemble selon la Revendication 1 ou 2, ***caractérisé en ce que*** chaque appareil de bronzage intégral (1, 2, 56-59, 80-92) présente un élément formant couchette (11 ; 21) et au moins un élément formant paroi (12, 13 ; 22, 23), ainsi qu'un élément formant plafond (14 ; 24) placé parallèlement à l'élément formant couchette (11 ; 21), et ***en ce qu'***au moins un élément d'entrée (15, 16 ; 25, 26) est placé entre l'élément formant couchette (11 ; 21) et l'élément formant plafond (14 ; 24).

4. Ensemble selon l'une quelconque des Revendications 1 à 3, ***caractérisé en ce que*** sont prévus contigus à l'élément formant couchette (11 ; 21), un élément d'entrée inférieur vertical (15 ; 25) et un élément d'entrée supérieur (16 ; 26), éléments qui sont placés mobiles sur un logement commun (3, 55) entre une position de service et une position d'ouverture.

5. Ensemble selon la Revendication 4, ***caractérisé en ce que*** l'élément d'entrée supérieur (16 ; 26), qui est monté pivotant sur l'élément d'entrée inférieur (15 ; 25), est tenu incliné dans la position de service entre l'élément d'entrée inférieur (15 ; 25) et l'élément formant plafond (14 ; 24), et ***en ce que***, pour la position d'ouverture, l'élément d'entrée supérieur (16 ; 26) pivoté en position verticale est mobile, conjointement avec l'élément d'entrée inférieur (15 ; 25), dans la direction verticale sur le logement (3, 55).

6. Ensemble selon l'une quelconque des Revendications 3 à 5, ***caractérisé en ce que*** l'élément formant plafond (14, 24) est monté mobile entre une position de service horizontale et une position d'ouverture.

7. Ensemble selon l'une quelconque des Revendications 3 à 6, ***caractérisé en ce que***, en relation de symétrie spéculaire par rapport aux éléments d'entrée (15, 16 ; 25, 26) dans la position de service, il est prévu sur la paroi de logement opposée respective (5, 7) un élément de paroi inférieur (12 ; 22) vertical et un élément de paroi supérieur incliné (13 ; 23), et ***en ce que*** l'élément formant plafond (14 ; 24) forme, avec l'élément de paroi incliné (13 ; 23) et l'élément d'entrée incliné (16 ; 26), un dispositif de type capot partiellement relevable.

8. Ensemble selon l'une quelconque des Revendications précédentes, ***caractérisé en ce que*** le logement commun (3) pour des appareils de bronzage intégral inférieur et supérieur (1 ; 2) en deux étages est conformé à la manière d'un placard, qui présente en coupe verticale la forme d'un S stylisé, et ***en ce que*** les ouvertures d'entrée (10 ; 20) sont placées chacune du côté concave des courbes du S.

9. Ensemble selon la Revendication 8, ***caractérisé en ce que*** le logement commun (3) présente un socle (4) côté plancher, un fond intermédiaire (6) entre les appareils de bronzage intégral inférieur et supérieur (1 ; 2), et un plafond (8) de logement, ***en ce que*** les éléments de paroi (12, 13) de l'appareil de bronzage intégral inférieur (1) peuvent se fixer sur la paroi (5) du logement et les éléments de paroi (22, 23) de l'appareil de bronzage intégral supérieur (2) peuvent se fixer sur la paroi (7) du logement.

10. Ensemble selon la Revendication 8 ou 9, ***caractérisé en ce que*** les éléments d'entrée (15, 16) de l'appareil de bronzage intégral inférieur (1) sont mobiles, pour la position d'ouverture, jusqu'au plafond (8) du logement sur la face extérieure de la paroi (7) du logement, et les éléments d'entrée (25, 26) de l'appareil de bronzage intégral supérieur (2) sont mobiles dans un caisson (29) du plafond (8) du logement.

11. Ensemble selon l'une quelconque des Revendications 8 à 10, ***caractérisé en ce qu***'un évidement (18 ; 28) est formé sur le fond intermédiaire (6) et sur le plafond (8) du logement pour recevoir l'élément formant plafond (14 ; 24) relevé.

12. Ensemble selon l'une quelconque des Revendications précédentes, ***caractérisé en ce que***, pour les appareils de bronzage intégral (1 ; 2, 56-59, 80-92), il est prévu une gaine d'air frais commune (50), qui alimente les chambres de bronzage (17 ; 27) en air frais.

13. Ensemble selon la Revendication 12, ***caractérisé en ce que***, pour l'air frais, une ouverture d'aspiration (53) côté fond et une évacuation d'air (54) côté plafond sont prévues dans le logement commun (3, 55).

14. Ensemble selon l'une quelconque des Revendications 12 ou 13, ***caractérisé en ce que***, dans le cas de deux appareils de bronzage intégral (1, 2) disposés sur deux étages, la gaine d'air frais commune (50) s'étend le long de la section verticale en S du logement commun (3).

15. Ensemble selon l'une quelconque des Revendications 2 à 14, ***caractérisé en ce qu***'une cabine de déshabillage (31) pour l'appareil de bronzage intégrai inférieur (1) est placée contiguë à la paroi (7) du logement et à l'ouverture d'entrée (10), et une cabine de déshabillage (32) pour l'appareil de bronzage intégrai supérieur (2) est placée contiguë à la paroi (5) du logement et à l'ouverture d'entrée (20), et ***en ce qu***'un podium (61) accessible par l'intermédiaire d'un escalier (62) est prévu comme dispositif d'accès (60).

16. Ensemble selon la Revendication 15, ***caractérisé en ce que*** des unités destinées au fonctionnement des appareils de bronzage intégral (1, 2), comme des appareils de climatisation et des appareils amont, sont placés dans le podium (61).

17. Ensemble selon l'une quelconque des Revendications 15 ou 16, ***caractérisé en ce que*** le logement commun (3) est préfabriqué, sous la forme d'une unité (30) de type conteneur, avec les appareils de bronzage intégral à plusieurs étages (1, 2), les cabines de déshabillage (31, 32) et le dispositif d'accès (60).

18. Ensemble selon la Revendication 17, ***caractérisé en ce que*** l'unité de type conteneur (30) présente un canal d'évacuation d'air (52) et ***en ce que*** chaque cabine de déshabillage (31 ; 32) est accessible par une porte séparée (41 ; 42).

19. Ensemble selon la Revendication 18, ***caractérisé en ce que*** le canal d'évacuation d'air (52) est placé dans la cabine de déshabillage (31) pour l'appareil de bronzage intégral inférieur (1), ***en ce que*** chaque cabine de déshabillage (31, 32) est munie d'un vestiaire (45 ; 46) et d'un miroir (43 ; 44) et ***en ce que*** les portes (41 ; 42) des cabines de déshabillage (31 ; 32) sont conformées en portes coulissantes de faible encombrement.

20. Ensemble selon l'une quelconque des Revendications précédentes 1 à 7, 12, 13, ***caractérisé en ce que*** dans le logement commun (55), qui, en coupe verticale, est conformé en méandre, il est prévu, au-dessus de deux appareils de bronzage intégral (56, 57), un troisième appareil de bronzage intégral (58), qui est accessible par l'intermédiaire d'un autre dispositif d'accès et d'une cabine de déshabillage séparée.

21. Ensemble selon l'une quelconque des Revendications précédentes 1 à 7, 12, 13, ***caractérisé en ce que*** plusieurs appareils de bronzage intégral (59...n) sont placés superposés les uns aux autres ou décalés dans la direction verticale et ***en ce que*** le dispositif d'accès et/ou les appareils de bronzage intégral (59...n) sont mobiles.

22. Ensemble selon l'une quelconque des Revendications précédentes 1 à 7, 12, 13, ***caractérisé en ce que*** plusieurs appareils de bronzage intégral (80, 81, 82 ; 83-90 ; 91, 92) sont agencés en une unité (70) et ***en ce que***, pour surmonter la différence de hauteur entre un sol et les ouvertures d'entrée, toute l'unité (70) et/ou les divers appareils de bronzage intégral (80, 81, 82 ; 83-90 ; 91, 92) peuvent être déplacés.

23. Ensemble selon l'une quelconque des Revendications précédentes 1 à 7, ***caractérisé en ce que*** plusieurs appareils de bronzage intégral (80, 81, 82 ; 83-90 ; 91, 92) sont agencés en une unité (70) et ***en ce que***, pour surmonter la différence de hauteur entre un plancher et les ouvertures d'entrée, toute l'unité (70) et/ou les divers appareils de bronzage intégral (80, 81, 82 ; 83-90 ; 91, 92) peuvent être déplacés.

24. Ensemble selon la Revendication 23, ***caractérisé en ce que*** l'unité (70) présente des appareils de bronzage intégrai superposés (80, 81, 82), qui peuvent être déplacés à la manière d'ascenseurs.

25. Ensemble selon la Revendication 23, ***caractérisé en ce que*** l'unité (70) présente des appareils de bronzage intégral superposés et juxtaposés (83-90), qui peuvent être déplacés à la manière d'un pater-noster.

26. Ensemble selon la Revendication 23, ***caractérisé en ce que***, dans l'unité (70), deux appareils de bronzage intégral (91, 92), qui sont placés l'un au-dessus de l'autre en position de service, peuvent être déplacés en cercle.

27. Ensemble selon la Revendication 26, ***caractérisé en ce que*** les appareils de bronzage intégrai (91, 92) sont montés sur un levier (94) de manière rotative autour de leurs axes longitudinaux (96, 97) et ***en ce que*** l'axe de rotation (95) du levier (94) est placé au milieu entre les deux appareils de bronzage intégral (91, 92).
